# EUROPEAN PATENT APPLICATION

(11) **EP 4 300 265 A1**
(43) Date of publication of application: **03.01.2024**
(21) Application number: 22382610.8
(22) Date of filing: 29.06.2022
(51) Int. Cl.: G06F 3/01, A61B 5/372, A61B 5/00, G06V 10/00, G06V 10/764, G06V 10/82

(54) **METHOD, SYSTEM AND COMPUTER PROGRAMS FOR THE AUTOMATIC LABELING OF IMAGES FOR DEFECT DETECTION**

(71) Applicant: Copysan Communicaciones, SL, 31192 Mutilva (ES)
(72) Inventor: SANTOS OLLOQUI, José, 31192 Mutilva (ES)
(74) Representative: Torner, Juncosa I Associats, SL

(57) **Abstract**

A computer implemented method, a system and computer program for the automatic labeling of images for defect detection. The method comprises storing acquired brain signals of a user, said brain signals being acquired using electrodes on the scalp of the user and in response to different motor imagery actions executed by the user, each one of the actions being executed for a while and as a result of the user having detected a defect in an image, or slice of an image, displayed on a visual stimuli screen; applying a filtering technique on the stored brain signals; classifying the filtered brain signals using a convolutional neural network; labelling the coordinates of the detected defects using weights of the convolutional neural network, providing an automatic labelling system; training an object detection model using the automatic labelling system, said model being usable for real time inference of image defects.

## Description

### Technical Field

The present invention relates to a method, system and computer program for performing an automatic labeling of images for defect detection. In particular, the invention relates to the detection of the defects based on motor imagery actions performed by a user.

### Background of the Invention

The brain processes any information by means of neurons that use electrical and chemical signals to communicate by releasing and receiving neurotransmitters. The neural activity in the human brain is an electrical change. In performing these functions, it produces complicated and complex biopotential signals. These signals can be recorded using different methods. Studying these electrical signals is vital to understanding the neurophysiological behavior of the brain. A number of techniques are used to study brain activities. Functional magnetic resonance imaging (fMRI), Functional Near-Infrared Spectroscopy (firs), and Electroencephalography (EEG) recordings are widely used techniques. The EEG signal is measured by placing multiple electrodes on the scalp that measure the current flow from neurons. These records, called electroencephalogram (EEG), contain a lot of information related to the work of the brain and other organs of the body. The objective is to capture signals of interest and reject the rest from the data extracted from the brain. The data may include relevant and irrelevant information of interest.

The brain signals usually contain signals varying from 0.5Hz to 100Hz frequencies. This is classified into 5 classes based on frequencies with each having distinct functions. The frequency bands; Delta, Theta, Alpha, Beta, and Gamma, are also called brain rhythms. Brain rhythms have been investigated over decades and a few characteristic behaviors of these brain rhythms have been established.

Classification of signals based on frequency and corresponding functions
- Gama (Signal frequency = 32 - 100Hz): Gamma waves are the higher frequency waves, ranging from 30 to onwards. Gamma waves are considered to play a complex role in brain functionality, such as combining information from two different sensory inputs. It is also used to confirm certain brain diseases. Some of the functions associated are learning, problem solving, cognitive processing, heightened perception
- Beta (Signal Frequency = 13 - 32Hz): Beta waves lie in the range of 13-32 Hz of frequency. Beta waves have been associated with active thinking, anxious, high alert, and focus of the brain. Some of the functions associated are awake, alert consciousness, thinking, excitement.
- Alpha (Signal Frequency = 8 - 13Hz): Alpha waves perhaps are the most widely investigated waves in EEG studies. Alpha waves were introduced by Berger in 1929. They lie in a range from 8 to 14 Hz. Alpha waves usually appear on the occipital lobe of the brain. Alpha waves are the most common indication of a relaxing state of mind and are also linked to closing eyes. Any sign of anxiety or attention reduces the alpha waves. Example of an associated function is Physically and mentally relaxed
- Theta (Signal Frequency = 4 - 8Hz): Theta waves were introduced by Dovey and Wolter, ranging from 4 to 8 Hz in frequency. Theta waves are linked to drowsiness and deep meditation. Some of the functions associated are creativity, insight, deep states, dreams, reduced consciousness, deep meditation.
- Delta (Signal Frequency = 0.5 - 4 Hz): Delta waves were first introduced by Walter in 1936, it ranges from 0.1 (or 0.5) to 4 Hz in frequency. Delta waves are usually observed in deep sleep. Since the delta wave is the low-frequency wave, it is easily confused by the movement artefact, due to similar nature. Delta waves have also been linked to continuous attention tasks. Some of the functions associated are deep sleep, repair, loss of bodily awareness.

### Description of the Invention

Present invention proposes, according to one aspect, a computer implemented method for the automatic labeling of images for defect detection. The method comprises: storing acquired brain signals of a user, said brain signals being acquired using a set of electrodes arranged throughout the scalp of the user and in response to different motor imagery actions performed by the user, each one of the motor imagery actions being executed during a given period of time as a result of the user having detected a defect in an image, or slice of an image, displayed on a visual stimuli screen; applying one or more filtering techniques on the stored brain signals; classifying the filtered brain signals using at least one convolutional neural network; labelling the coordinates of the detected defects using weights of the convolutional neural network, providing an automatic labelling system as a result; and training an object detection model using the provided automatic labelling system, said trained object detection model being usable for real-time defect inference.

Present invention also proposes, according to another aspect, a system for the automatic labeling of images for defect detection. The system comprises: a visual stimuli screen; a set of electrodes configured to be arranged throughout the scalp of a user and configured to acquire brain signals of the user in response to different motor imagery actions performed by the user, each one of the motor imagery actions being executed during a given period of time as a result of the user having detected a defect in an image, or slice of an image, displayed on said visual stimuli screen; and a processing unit, comprising a memory and at least one processor, configured to: store the acquired brain signals; apply one or more filtering techniques on the stored brain signals; classify the filtered brain signals using at least one convolutional neural network; label the coordinates of the detected defects using weights of the convolutional neural network, providing an automatic labelling system as a result; and train an object detection model using the provided automatic labelling system, said trained object detection model being usable for real-time defect inference.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

In some embodiments, the set of electrodes are placed on a helmet.

In some embodiments, the different motor imagery actions comprises one or more of the following actions: closing the left hand, closing the right hand, performing a dorsiflexion on left foot or performing a dorsiflexion on the right foot, among others.

In some embodiments, the filtering techniques comprise using a notch filter and/or a bandpass filter, among others.

### Brief Description of the Drawings

The previous and other advantages and features will be more fully understood from the following detailed description of embodiments, with reference to the attached figures, which must be considered in an illustrative and non-limiting manner, in which:
Fig. 1 is a flowchart of a method for the automatic labeling of images for defect detection, according to an embodiment of the present invention.
Fig. 2 is a flowchart illustrating the detailed implementation of the defect detection, according to an embodiment.
Figs 3A-3D show some examples of the images used in Fig. 2. Fig. 3A illustrates the full image; Fig. 3B illustrates a vertical slice of the image of Fig. 3A; Fig. 3C and 3D are some examples of horizontal slices of the image.

### Detailed Description of the Invention and of Preferred Embodiments

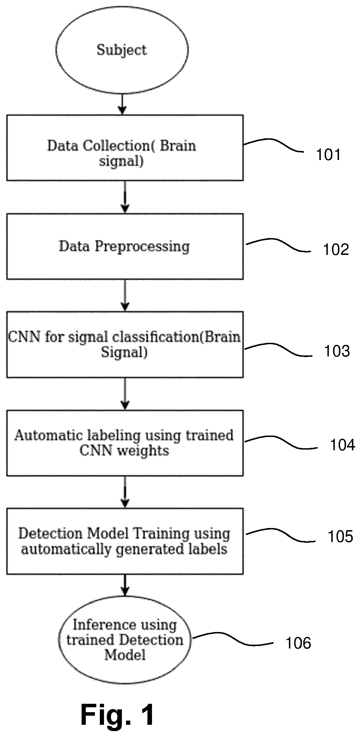

Fig. 1 illustrates an embodiment of the proposed method. Particularly, to start the method, the subject/user is allowed to sit in an environment, in which the user is able to be in a relaxed position, and electrodes arranged on his/her scalp.

The electrodes are particularly comprised on a brain computer interface (BCI) helmet. The electrodes capture the brain signals and send the captured signals to a computing device or processing unit. In some embodiments, the OpenBCI Cyton Board is used, which is an Arduino-compatible, 8-channel neural interface with a 32-bit processor. At its core, the OpenBCI Cyton Board implements a PIC32MX250F128B microcontroller, giving it lots of local memory and fast processing speeds. The OpenBCI Cyton Board can be used to sample brain activity (EEG), muscle activity (EMG), and heart activity (ECG). The board communicates wirelessly to a computer via the OpenBCI USB dongle using RFDuino radio modules. It can also communicate wirelessly to any mobile device or tablet compatible with Bluetooth Low Energy (BLE).

The OpenBCI data is streamed from the helmet to the system through the Lab Streaming Layer (LSL) netwrork protocol. LSL is a networking system for real time streaming, recording, and analysis of time-series data. LSL can be used to connect OpenBCI to applications that can record, analyze, and manipulate the data, such as Matlab, NeuroPype, BCILAB, and others.

Additionally, the name of the electrode position on the brain is labeled as character followed by a number to identify the part of the brain. The characters are Fop for pre-frontal, F for frontal, P for parietal, T for temporal, O for occipital, and C for the central lobe of the brain. A few in between two landmarks are named with two characters, such as AF, between Fp and

F and FC, between F and C. Position electrodes on different parts of the helmet according to brain functioning. The used formation of electrodes is:

**Table 1: Channel positions.**

| Electrode/Channel | 0 | 1 | 2 | 3 | 4 | 5 | 6 | 7 | SRB2 | BIAS |
|---|---|---|---|---|---|---|---|---|---|---|
| Location | Cz | C4 | C3 | FC2 | T3 | Pz | FC1 | T4 | A2 | A1 |

The motor cortex is the region of the cerebral cortex involved in the planning, control, and execution of voluntary movement. In the data preparation task, a visual stimuli will be shown in the visual stimuli screen and the user has to perform the motion action based on the stimulus. In that kind of situation, the signals of interest will be in the area of the motor cortex based on that the electrode positions were selected.

Dry electrodes are preferably used. Dry electrodes consist of a single metal that acts as a conductor between the skin and the electrode. This material is usually stainless steel. The dry electrode is fast and easy to apply while compared with wet electrodes. But if the electrodes are properly positioned, with firm contact between the skin and the electrode, reliable levels of spectral EEG can be measured with or without per-amplification. That is, the fact that it is an active or passive electrode does not seem to add additional noise in the EEG measurements. In addition, with the dry electrodes, it is possible to carry out the experiments that were previously carried out inside a closed environment to the outside. In other words, EEG measurements can be taken in a real-world environment.

Following with the explanation of Fig. 1, at step 101, the brain or EEG data is collected from the user using the electrodes and the acquired brain or EEG signals undergo a signal preprocessing process (step 102) to remove any artefact (e.g. motion, muscular, ocular, and cardiac) present in the signals. In a particular embodiment, to preprocess the signals a notch and a bandpass filter are used. A notch filter is a band-stop filter with a narrow stopband. The notch filter is applied with notch frequency 50Hz to the interference in the signal(s).The quality factor is chosen as 20, which calculates the quality factor of a notch filter circuit. A band-pass filter or bandpass filter (BPF) is a device that passes frequencies within a certain range and rejects (attenuates) frequencies outside that range.

At step 103, the preprocessed or filtered signals are used as the input of a convolution neural network for classification thereof based on the images shown by the visual stimuli screen. The convolutional neural network can be optimized using an Adam optimizer and the loss calculated using categorical crossentropy. The network is converged, and the best weights saved to be latter used for the automatic labelling.

In general, the classification of EEG signals has traditionally relied upon manual feature extractors and classifiers. The deep learning architectures although newly introduced, have superseded their performances. The AttentionEEGNet is a deep learning neural network architecture which is developed based on the EEGNet. In the EEGNet a Convolution Block Attention Module (CBAM), an extra convolution and L2 regularizer is introduced.

CBAM, Sanghyun Woo et.al (2018), contains two sequential sub-modules called the Channel Attention Module (CAM) and the Spatial Attention Module (SAM), which are applied in that particular order. The authors of the paper point out that CBAM is applied at every convolutional block in deep networks to get subsequent "Refined Feature Maps" from the "Input Intermediate Feature Maps". Spatial refers to the domain space encapsulated within each feature map. Spatial attention represents the attention mechanism/attention mask on the feature map, or a single cross-sectional slice of the tensor. Channels are essentially the feature maps stacked in a tensor, where each cross-sectional slice is basically a feature map of dimension (h × w). Usually in convolutional layers, the trainable weights making up the filters learn generally small values (close to zero), thus similar feature maps with many appearing to be copies of one another are observed. The channel attention essentially provides a weight for each channel and thus enhances those particular channels which are most contributing towards learning and thus boosts the overall model performance.

In the AttentionEgg, there are 2 convolutions, 1 depthwise convolution ,1 separable convolution, 1 fully connected layer and a softmax classification layer. The batch normalization, CBAM block , pooling layer and dropout is added in between the layers. The first convolution layer uses an 8 filter with I2 regularizer followed by batch normalization and CBAM Block. The depthwise convolution with depth 2 is followed by that, which is followed by batch normalization, CBAM block, Average pooling and dropout. The Separable convolution with filter size 16 is followed by that, which is followed by batch normalization CBAM block, Average pooling and dropout. The Second convolution layer with 32 filters and L2 regularizer is followed by that, which is then connected to the batch normalization, CBAM block, Average pooling and dropout. The output from the convolution layer is flattened and fetched into the fully connected layer with 256 neurons and the final classification layer using softmax contains 5 neurons which classify the input signal into respective actions.

At step 104, the cited best weights are used for labelling the coordinates of the detected defects. The labelled defects are then used, at step 105, for training an object detection model which can be used for real-time defect inference (step 106).

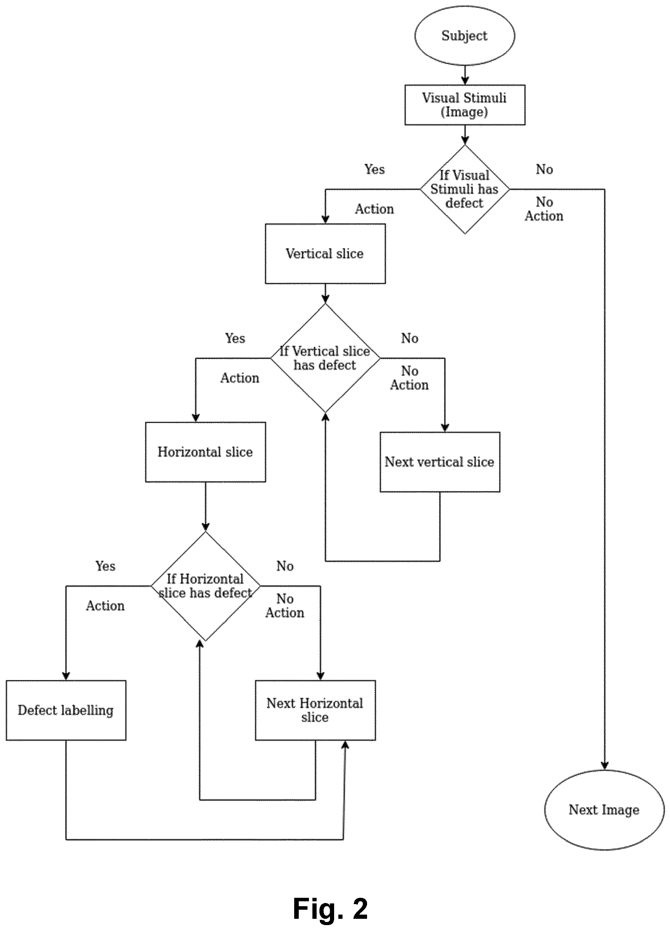

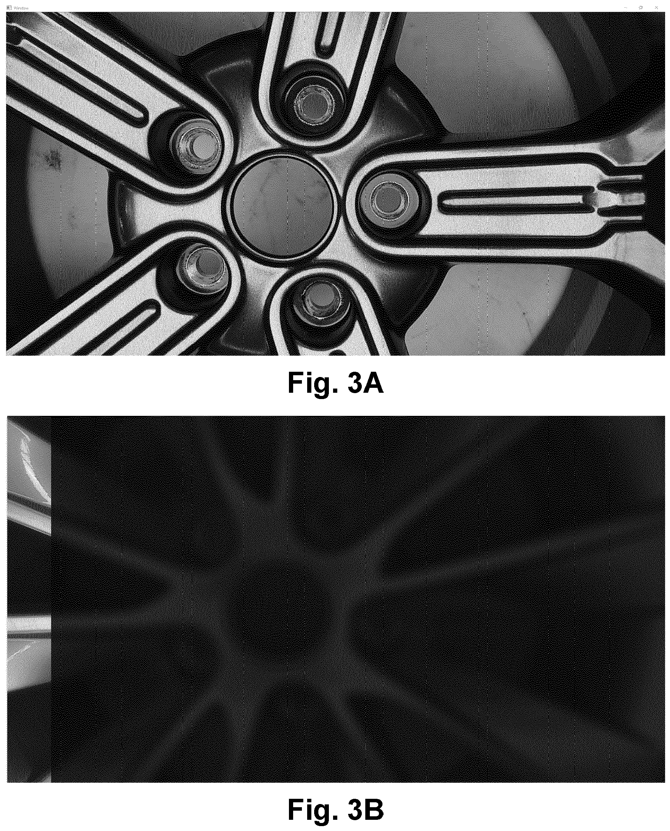

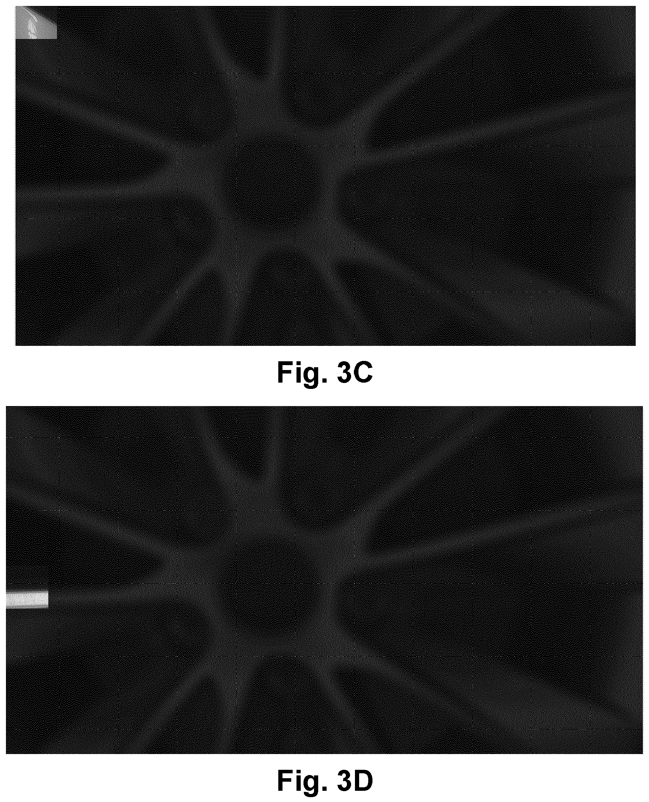

With reference now to Figs. 2 and 3, therein an embodiment of the defect detection performance is illustrated. As said, in order to do the automatic labelling, the trained weights from the convolutional neural network, particularly the AttentionEGGNet, are used. According to this embodiment, the electrodes are connected to the user and made her/him relax. The visual stimuli will be shown in the whole screen and the user has to do the motor imagery actions based on the visual stimuli. If the user finds any defect (s)he has to do the assigned actions, for example:

| Actions | Defects |
|---|---|
| closing the left hand (LCH) | Notch |
| closing the right hand (RCH) | line |
| Left Foot Dorsiflexion (LDF) | point |
| Right Foot Dorsiflexion (RDF) | dent |
| Norm (No action) | Skip to the next section |

The system first provides to the user an input image. If the user identifies any defects in the image, then the system goes for slicing the image vertically. Then, if the user identifies any defects in the vertical slice the system goes for slicing the image horizontally. If no defects are identified by the user, the system slices another horizontal slice or goes to the next image, otherwise, if a defect is identified the system returns the accurate coordinates. That is, according to this particular embodiment, the order of the slicing will be: Full Image --> Vertical Slice --> Horizontal Slice.

The process will be performed till the last segment of the image is considered. The coordinates of the defects location will be saved for creating labels, which will be used for training data.

The label files will be saved based on the motor imagery actions given by the user. The label file will be preprocessed in order to change to the prescribed format of the object detection model.

In a particular embodiment, the YOLO V3-Tiny algorithm is used as the object detection model. The Tiny YOLOv3 is used for the real-time detection. The Tiny YOLOv3 trunk feature extraction network has seven convolution layers with 3 × 3 convolution kernels and one convolution layer with 1 × 1 convolution kernels, six layers of maxpooling are used to reduce the parameters. The object is predicted by using a two-scale prediction network with the output feature map of 13 × 13 and 26×26. In the prediction network, the Tiny YOLOv3 uses the upsampling to extract feature and strengthen the feature fusion. In the figure given below, the 13x13 feature map passes the convolution layer and upsampling layer. This turns the 13×13×12 feature map into 26 × 26 × 256. The feature map of 26 × 26 also is taken from the earlier in the network and merged with the upsampling feature by concatenation. Finally, the output feature map of 26 × 26 is formed. Tiny YOLOv3 divides the input image into N x N grids and predicts the bounding boxes within each grid cell, and the target is detected. Finally, the bounding boxes and confidence for each classification of targets are proposed.

The labels of the image annotated using the automatic annotation using the brain signals are converted into the format of YOLO. Likewise, the data is augmented using rotation, applying noise and enhancement filters. The data augmentation is used to increase the number of images which will help the system to improve the accuracy of the detection model.

The automatically labeled data has been trained with augmented images and their corresponding labels with a learning rate of 0.001 in which the base weights have been chosen from the coco trained weights. The data has been trained for 100000 iterations which has given good performance and better time complexity. The model has converged in the data.

Various aspects of the proposed method, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with image processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

Those skilled in the art will recognize that the present teachings are amenable to a variety of modifications and/or enhancements. For example, although the implementation of various components described herein may be embodied in a hardware device, it may also be implemented as a software only solution-e.g., an installation on an existing server. In addition, image processing as disclosed herein may be implemented as a firmware, firmware/software combination, firmware/hardware combination, or a hardware/firmware/software combination.

The scope of the present invention is defined in the following set of claims.

## Claims

1. A computer implemented method for the automatic labeling of images for defect detection, comprising:
storing acquired brain signals of a user, said brain signals being acquired using a set of electrodes arranged throughout the scalp of the user and in response to different motor imagery actions performed by the user, each one of the motor imagery actions being executed during a given period of time as a result of the user having detected a defect in an image, or slice of an image, displayed on a visual stimuli screen;
applying one or more filtering techniques on the stored brain signals;
classifying the filtered brain signals using a convolutional neural network;
labelling the coordinates of the detected defects using weights of the convolutional neural network, providing an automatic labelling system as a result;
training an object detection model using the provided automatic labelling system, said trained object detection model being usable for real-time defect inference.

2. The method of claim 1, wherein the different motor imagery actions comprises one or more of the following actions: closing the left hand, closing the right hand, performing a dorsiflexion on left foot or performing a dorsiflexion on the right foot.

3. The method of any one of the previous claims, wherein the filtering techniques comprise using a notch filter and a bandpass filter.

4. A system for the automatic labeling of images for defect detection, comprising:
a visual stimuli screen;
a set of electrodes configured to be arranged throughout the scalp of a user and configured to acquire brain signals of the user in response to different motor imagery actions performed by the user, each one of the motor imagery actions being executed during a given period of time as a result of the user having detected a defect in an image, or slice of an image, displayed on said visual stimuli screen;
a processing unit, comprising a memory and at least one processor, configured to:
- store the acquired brain signals;
- apply one or more filtering techniques on the stored brain signals;
- classify the filtered brain signals using a convolutional neural network;
- label the coordinates of the detected defects using weights of the convolutional neural network, providing an automatic labelling system as a result; and
- train an object detection model using the provided automatic labelling system, said trained object detection model being usable for real-time defect inference.

5. The system of claim 4, wherein the set of electrodes are placed on a helmet.

6. The system of claim 4 or 5, wherein the different motor imagery actions comprises one or more of the following actions: closing the left hand, closing the right hand, performing a dorsiflexion on left foot or performing a dorsiflexion on the right foot.

7. The system of any one of the previous claims 4 to 6, wherein the filtering techniques comprise a notch filter and a bandpass filter.

8. A non-transitory computer readable medium comprising code instructions that when executed by a computer system implement the method of any one of claims 1 to 3.
